(19) Europäisches Patentamt
European Patent Office
Office européen des brevets

(11) Publication number: **0 305 567**
**A1**

(12) **EUROPEAN PATENT APPLICATION**

(21) Application number: **87112800.5**

(22) Date of filing: **02.09.87**

(51) Int. Cl.$^4$: **C07C 37/20 , C07C 39/16 , C07C 39/367 , C07C 43/205**

(43) Date of publication of application: **08.03.89 Bulletin 89/10**

(84) Designated Contracting States: **CH DE ES FR GB IT LI NL**

(71) Applicant: **THE DOW CHEMICAL COMPANY**
**2030 Dow Center Abbott Road P.O. Box 1967**
**Midland Michigan 48640-1967(US)**

(72) Inventor: **Chang, Kuo Yuan**
**2708 Mt. Vernon**
**Midland Michigan 48640(US)**

(74) Representative: **Sternagel, Hans-Günther, Dr. et al**
**Patentanwälte Dr. Michael Hann Dr. H.-G. Sternagel Sander Aue 30**
**D-5060 Bergisch Gladbach 2(DE)**

(54) **Preparation of trisphenol methanes.**

(57) Tris-(p-hydroxy-disubstituted phenyl) methanes of the formula

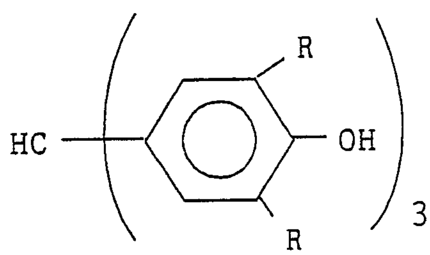

wherein each R independently is hydrogen, halogen, or alkoxy, aryloxy or alkyl of up to 10 carbon atoms is prepared by contacting 2,6-disubstituted phenols with salicylaldehyde.

**EP 0 305 567 A1**

## PREPARATION OF TRISPHENOL METHANES

The present invention relates to a process for the preparation of trisphenol methanes.

Certain pharmaceutical hydroxyl-containing triaryl methanes and their preparation are disclosed in US Re. 31,467. Other generic references to triaryl methane compounds include US 877,053; 3,449,418; 3,739,000; and 4,048,200. It is generally well-known that trisphenolics can be prepared by condensing an alkoxy-substituted aromatic aldehyde or an alkoxy-substituted aromatic ketone with an excess of a substituted or unsubstituted phenol. See, e.g., US 2,116,827; 3,787,451; and 4,394,496. US 3,579,542 teaches the preparation of 4,4′,4″-trihydroxytriphenylmethyl methane by condensing p-hydroxyacetophenone with phenol using a metal halide or acid catalyst. It is also known to react salicylaldehyde with certain phenolic compounds to obtain 4,4′,2″-trihydroxyphenyl methanes. See, e.g., Beilstein EII6, pp. 1111-2; EIV6, pp. 7630-1; and EIV6, pp. 7643-4.

It is known to prepare 4,4′,4″-methylidyne tris-(2,6-dimethylphenol) by reacting ethyl orthoformate (triethoxy methane) with the magnesium bromide Grignard reagent of 2,6-dimethylphenol. Chemical Abstracts 68:12811g. Said method employs relatively expensive starting materials, and requires special care since Grignard reagents react rapidly with both water and oxygen.

Heretofore, 4,4′,4″-methylidyne tris-(2,6-dimethylphenol) and related tris(p-hydroxyphenyl) methanes have not been prepared from the relatively inexpensive salicylaldehyde and its derivatives.

The present invention concerns a process characterized by contacting a 2,6-disubstituted phenol with salicylaldehyde under reaction conditions such that there is formed a product represented by the formula

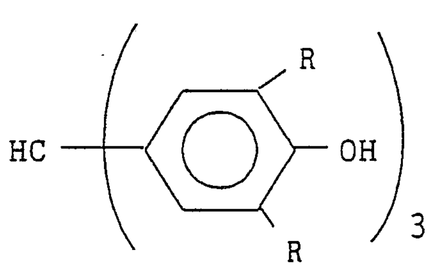

wherein each R independently is hydrogen, halogen or alkoxy, aryloxy or alkyl of up to 10 carbon atoms.

Surprisingly, the tris(p-hydroxyphenyl) product is obtained without using a p-hydroxyphenyl ketone or aldehyde as a starting material. The tris-(4-hydroxy-3,5-disubstituted phenyl) methanes are useful chemical intermediates, and can be converted to tris epoxy resins using known methods.

The process of the present invention advantageously employs a 2,6-disubstituted phenol and salicylaldehyde.

A 2,6-disubstituted phenol desirably having a boiling point higher than the boiling point of phenol is advantageously employed in the process of the present invention. The 2,6-disubstituted phenol can have a wide variety of substitutents so long as they do not prevent the reaction. Examples of typical substitutents include hydrogen, halogen, hydrocarbyl, substituted hydrocarbyl, alkoxy and aryloxy of up to 10 carbon atoms. Preferred substitutents include alkyl of up to 10 carbon atoms. More preferred substitutents are n-alkyl. The most preferred 2,6-disubstituted phenol is 2,6-dimethylphenol. Mixtures of these phenols can be employed, and the 2- and 6-substitutents can be different. The substituents preferably are such that phenol can be easily separated from the reaction mixture using known separation techniques, such as distillation.

The 2,6-disubstituted phenol can be employed in any amount which will give the desired product. Typically, the molar ratio of the 2,6-disubstituted phenol to salicylaldehyde is from 3 to 10, and preferably is from 4 to 6.

The contacting of salicylaldehyde and the 2,6-disubstituted phenol can occur under any set of reaction conditions which give the desired product. Preferably, the reactants are contacted in a distillation column reactor under any combination of conditions sufficient to distill the phenol overhead. Typical temperatures range from 42°C to 120°C and preferably are from 60°C to 90°C. The pressure typically is from 2 mm Hg to 100 mm Hg, and preferably is from 6 mm Hg to 30 mm Hg.

A catalyst is optionally employed in the process of the present invention, and can be any material which catalyzes the reaction. Examples of typical catalysts include acid catalysts such as mineral acids, organic acids such as p-toluene sulfonic acid, and solid acids such as acidic ion-exchange resins. Mixtures of catalysts can be employed. When employed, the catalyst is employed in a catalytic amount. Preferably, the catalyst is employed in an amount such that the ratio of acid equivalents to starting salicylaldehyde is from 0.05 to 0.2.

When salicylaldehyde and a 2,6-disubstituted phenol are contacted as described hereinabove, a tris(4-hydroxy-3,5-disubstituted phenyl) methane is produced. Preferred product compounds are repre-

sented by the formula:

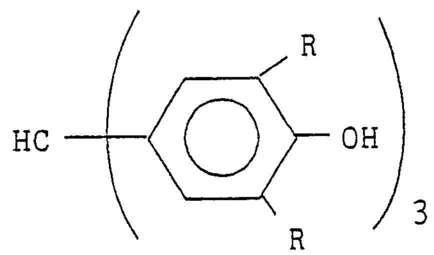

wherein each R independently is halo, hydrocarbyl or substituted hydrocarbyl. The R moieties are defined by the substitutents on the 2,6-disubstituted phenol.

The following example is given to illustrate the invention.

Example 1

A mixture of 188.8 g of 2,6-dimethylphenol and 69.5 g of salicylaldehyde was stirred and heated at 118° C in the presence of 3.0 g of toluene sulfonic acid monohydrate for 24 hours. Then, an additional 30.4 g of 2,6-dimethylphenol was added to the mixture, and the resulting mixture was distilled at a pressure of 180-200 mm Hg in a 20-plate distillation column having a diameter of one inch (2.5 cm) to remove 35 g of phenols overhead. The material in the distillation pot was poured into 600 ml of room temperature methanol. The methanol solution was seeded with an authentic 4,4′,4″-methylidyne tris(2,6-dimethylphenol) and was allowed to stand overnight. The resulting crystals were collected by filtration and were washed with fresh methanol and then dried in air to give 25 g of orange crystals having a nuclear magnetic resonance spectrum identical to that of an authentic sample.

**Claims**

1. A process characterized by contacting a 2,6-disubstituted phenol with salicylaldehyde under reaction conditions such that there is formed a product represented by the formula

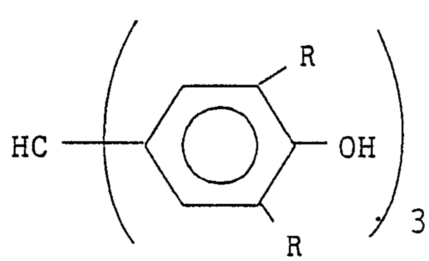

wherein each R independently is hydrogen, halogen or alkoxy, aryloxy or alkyl of up to 10 carbon atoms.

2. The process of Claim 1 wherein the contacting is conducted at a temperature of from 42° C to 120° C.

3. The process of Claim 1 wherein the contacting is conducted in the presence of a catalyst.

4. The process of Claim 1 wherein from 3 to 10 moles of the 2,6-disubstituted phenol are employed per mole of salicylaldehyde.

5. The process of Claim 1 wherein each R independently is an alkyl of up to 10 carbon atoms.

6. The process of Claim 5 wherein at least one R is methyl.

7. The process of Claim 6 wherein each R is methyl and the contacting is conducted under distillation reaction conditions in the presence of an acidic catalyst.

8. The process of Claim 7 wherein the catalyst is toluene sulfonic acid or a hydrate thereof.

9. The process of Claim 3 wherein the catalyst is an acidic catalyst.

10. The process of Claim 9 wherein the catalyst is toluene sulfonic acid or a hydrate thereof.

European Patent Office

# EUROPEAN SEARCH REPORT

Application Number

EP 87 11 2800

| DOCUMENTS CONSIDERED TO BE RELEVANT | | | |
|---|---|---|---|
| **Category** | **Citation of document with indication, where appropriate, of relevant passages** | **Relevant to claim** | **CLASSIFICATION OF THE APPLICATION (Int. Cl.4)** |
| A | HOUBEN-WEYL: "Metoden de Organischen Chemie", vol. 6/1C, Part 2, 1976, pages 1058-1974, Georg Thieme Press, Stuttgart<br>* page 1059, lines 11-18; page 1973, lines 15-29 *<br>--- | 1 | C 07 C 37/20<br>C 07 C 39/16<br>C 07 C 39/367<br>C 07 C 43/205 |
| A | AT-B- 289 093 (HOECHST)<br>* page 2, example * & US - A - 35 79542 (Cat. D)<br>--- | 1 | |
| A | DK-C- 56 408 (CHINOIN)<br>* examples *<br>--- | 1 | |
| A | LA CHIMICA E L'INDUSTRIA, vol. 49, no. 6, June 1967, pages 630-633, publ. Societa Anonima Ed. di Chimica, Milan, IT; B. CARDILLO: Reattività di sistemi nucleofili ambifunzionali. Reazione tra ortoformiato d'etile e sistemi fenolici"<br>* page 632, example 1 * & CHEMICAL ABSTRACTS, vol. 68, no. 3, 15th January 1960, abstract no. 12811g (Cat. D)<br>--- | 1 | **TECHNICAL FIELDS SEARCHED (Int. Cl.4)**<br>C 07 C 37/00<br>C 07 C 39/00 |
| A | US-A-4 152 341 (JONES)<br>* column 5, lines 17-47 * & US - E 31467 (Cat. D)<br>----- | 1 | |

The present search report has been drawn up for all claims

| Place of search | Date of completion of the search | Examiner |
|---|---|---|
| BERLIN | 18-04-1988 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)